# EUROPEAN PATENT SPECIFICATION

(11) **EP 1 926 503 B1**
(45) Date of publication and mention of the grant of the patent: **24.08.2011**
(21) Application number: 06786950.3
(22) Date of filing: 11.07.2006
(51) Int. Cl.: A61L 9/14, C11D 3/00

(54) **COMPOSITION AND METHOD FOR DEACTIVATING ALLERGENIC PROTEINS ON SURFACES**
ZUSAMMENSETZUNG UND VERFAHREN ZUR DEAKTIVIERUNG VON ALLERGENEN PROTEINEN AUF OBERFLÄCHEN
COMPOSITION ET PROCEDE POUR DESACTIVER DES PROTEINES ALLERGENES SUR DES SURFACES

(30) Priority: 12.07.2005 US 698348 P
(43) Date of publication of application: 04.06.2008
(73) Proprietor: STEPAN COMPANY, Northfield, IL 60093 (US)
(72) Inventor: TABOR, Rick, L., Glenview, IL 60025 (US); HARTLAGE, James, Chicago, IL 60611 (US); BERTHOLF, Daniel, Scott, Glenview, IL 60025 (US)
(74) Representative: Schnappauf, Georg
(86) International application number: PCT/US2006/026972
(87) International publication number: WO 2007/008938

(56) References cited:
- EP-A- 1 228 717
- WO-A-2004/078680
- GB-A- 1 472 084

## Description

### RELATED APPLICATIONS

This application claims priority to U.S. Provisional Application Serial No. 60/698,348, filed July 12, 2005, now abandoned, the disclosure of which is hereby incorporated by reference herein in its entirety.

### FIELD OF THE INVENTION

The presently descried technology relates generally to methods and compositions for deactivating the allergenic protein content of surfaces by treatment with an allergenic protein deactivating compound. In some embodiments, allergenic protein deactivating formulations of the present technology additionally contain antimicrobial agents to deactivate microorganisms on surfaces, in the air, or both.

### BACKGROUND OF THE INVENTION

Allergens are substances that trigger allergic reactions. Common cleaning methods are often insufficient to substantially neutralize and/or remove many of the allergens commonly present on various surfaces. As a result, various anti-allergen chemistries have been developed for use in various contexts.

Allergen neutralizers that are currently known include, for example, tannic acid and polyphenols, bleach, enzyme solutions and cyclodextrins. Tannic acid and polyphenols are inappropriate for many applications, such as for use on fabrics, because they are known to cause staining and are skin irritants. Chlorine bleach is commonly used against mold on hard surfaces such as tile, metal and plastics, but is inappropriate for use on colored fabrics because it removes coloration, and can cause skin irritation.

One commercial example of a formulation using cyclodextrin is Febreze® Allergen Reducer produced by Proctor & Gamble of Cincinnati, Ohio. The patents that are said to cover this product include one or more of U.S. Patent Nos. 6,146,621; 5,942,217; 5,939,060; 5,783,544; 5,714,137; 5,668,097; and 5,593,670. As an example, U.S. Patent No. 6,146,621 warns that the Proctor & Gamble formulation should not be applied to shiny surfaces including, e.g., chrome, glass, smooth vinyl, leather, shiny plastic, shiny wood, etc., because spotting and filming can more readily occur on those types of surfaces with the subject cyclodextrin-containing formulation. Additionally, the formulation is described as not being suitable for use on human skin, especially when an antimicrobial preservative is present in the composition, because skin irritation can occur.

Another example of an allergen reducing formulation is described in U.S. Patent Publication No. 20040007251, which provides for wipes used to remove an allergen from (among other things) hard surfaces, human skin and animal fur. The wipes are further described in U.S. Patent Publication No. 20040007251 as containing an additive, such as a lectin, a protease, and/or an enzyme inhibitor that can bind the allergen to the wipe such that the allergen can be removed from the surface. Patent Publication No. 20040007251 also provides that when the wipes contain a protease or an enzyme inhibitor, alone or in combination with a lectin, the protease or enzyme inhibitor may provide the additional benefit of chemically altering the allergen to reduce its allergenicity.

Another example of an allergen deactivating formulation is described in U.S. Patent No. 6,800,247, which describes a method for deactivating Dermatophagoides farinae (Der-f) and Dermatophagoides pteronyssinus (Der-p) dust mite allergens using a variety of deactivating agents. The list of deactivants includes polyquaternium 10 (which is described as being hydroxyethyl cellulose reacted with Polymer JR-125, a trimethyl ammonium epoxide compound), polyvinyl pyrrolidone and hexadecyl trimethyl ammonium chloride. The reference further provides that some of the deactivants are specific to the type of dust mite allergen being treated. For example, an effective Der-f allergen deactivant will not automatically work effectively as a Der-p allergen deactivant. The use of polyquaterniums based on cellulose (such as polyquaternium 10) is undesirable in certain applications because these types of polyquaterniums increase the wettability of textile surfaces and subsequently reduce the stain resistance of textiles to which they are applied due to their high degree of hydrophilicity.
WO 2004/078680 describes a method of reducing and/or neutralizing the allergenic protein content of natural rubber latex (NRL) articles by exposing the natural rubber latex from which the article is made or the final natural rubber latex to an allergenic protein reducing compound.

One commercial example of an allergen reducing enzymatic formula is a pet care product called Nature's Miracle^{®} Dander Remover & Body Deodorizer, produced by Pets'N People, Inc., a subsidiary of Eight In One Pet Products of Hauppauge, New York. This product is described as a non-toxic, biodegradable enzyme formula that is designed to be sprayed onto a pet's fur or skin and wiped off with a paper towel.

Other pet care products include Allerpet^{®} Dander Lotion and Simple Solution^{®} Allergy Relief Wipes. Allerpet^{®} Dander Lotion is produced by Farnam Companies, Inc., located in Phoenix, Arizona and is described as a non-toxic, odorless and safe solution that removes the saliva, dander and urine allergens from an animal's coat and skin. Allerpet^{®} Dander Lotion can be wiped or sprayed onto an animal and then rubbed or combed into the animal's fur. Simple Solution^{®} Allergy Relief Wipes are produced by Bramton Company of Dallas, Texas, and are described as pre-moistened cloths that contain a blend of conditioning agents and proteins to wipe away pet dander and loose hair. However, neither of these products is described as being suitable for use on other surfaces such as textiles or hard surfaces.

In light of the above, there exists a continued need for a low toxicity, low staining, mild anti-allergenic formulation which deactivates allergenic proteins on surfaces that is preferably non-irritating to humans and animals.

### BRIEF SUMMARY OF THE INVENTION

The present invention generally relates to deactivating allergenic proteins on various surfaces by treating them with an allergenic protein deactivating agent. The present technology can be useful, for example, in the following applications: sprays for upholstery or carpet, sprays for textiles, additives for dusting sprays, a pretreatment for newly manufactured carpet to deactivate allergenic proteins as the carpet absorbs or interacts with them, a treatment for ducts to deactivate allergenic proteins as they occur during air passage, an anti-allergenic treatment in combination with currently used biocides for mold-infested ducts, a treatment for allergen-reducing air filtration systems and home air filters, an additive for medicinal creams or lotions, a treatment for masks used by people to reduce exposure to allergens, a skin spray used by people to reduce exposure to allergens, or a treatment for allergen-producing pets.

As used herein, the terms "deactivating," "deactivation" and "deactivate" refer to rendering allergenic proteins inactive and/or ineffective with respect to allergenic impact inhumans or animals, and encompass both partial and total deactivation, as well as a reduction in or a prevention of allergenic impact. Allergen deactivation can be measured by immunoassay of the substances removed from a surface after treatment of the surface using the present technology.
In one aspect, the present invention provides an allergenic protein deactivating formulation comprising an allergenic protein deactivating compound comprising at least about 0.05% by weight of a quaternary amine salt mixture comprising: at least about 32% by weight n-alkyl (50% C14, 40% C12, 10% C16) dimethyl benzyl ammonium chloride; at least about 24% by weight n-octyl decyl dimethyl ammonium chloride; at least about 12% by weight di-n-octyl dimethyl ammonium chloride; and at least about 12% by weight di-n-decyl dimethyl ammonium chloride.
In another aspect, the present invention provides a method for deactivating allergenic proteins on a surface, in the air or on skin comprising the step of exposing the surface, air or skin to an allergenic protein deactivating formulation which comprises an allergenic protein deactivating compound comprising at least about 0.05% by weight of a quaternary amine salt mixture comprising: at least about 32% by weight n-alkyl (50% C14, 40% C12, 10% C16) dimethyl benzyl ammonium chloride; at least about 24% by weight n-octyl decyl dimethyl ammonium chloride; at least about 12% by weight di-n-octyl dimethyl ammonium chloride; and at least about 12% by weight di-n-decyl dimethyl ammonium chloride.
Preferred embodiments of the aspects of the present invention are set forth in claims 2 to 6 and 8 to 12.
Methods of exposing surfaces to an allergenic protein deactivating formulation of the present technology include, for example, spraying or wiping the surface with a dilute solution of the allergenic protein deactivating compound.

Solutions of the present technology are intended to be safe for contact with humans and animals, and provide deactivation of allergenic proteins on the surfaces to which they are applied. The solutions preferably contain a reduced level of particulate material as compared to various other conventional anti-allergenic formulations. More preferably, the solutions contain a low level of particulate material (i.e. the allergenic deactivating agent). Specifically, it is preferred that these solutions contain no more than about 5% by weight of particulate materials. More preferably, solutions contain about 1% or less of the particulate matter.

Surfaces on which the present technology may be used include textiles, leather, fur, non-wovens, and wood and other hard surfaces. In order to avoid streaking and filming on hard or smooth surfaces, it is desired that the concentration of the allergenic deactivating agent or blend of agents be less than about 5% by weight in the solvent, aqueous composition, or diluent used to deliver the agent or agents. Preferably, the concentration of the allergenic deactivating agent or blend of agents is less than about 2.5% by weight, less than about 2% by weight, or less than about 1.5% by weight. Most preferably, the concentration of the allergenic deactivating agent or blend of agents is about 1% by weight, or less than about 1% by weight Suitable amounts of an allergenic deactivating agent or blend of agents that are less than about 1% include, for example, concentrations up to about 0.5% by weight, or up to about 0.2% by weight.

The methods and compositions of the present technology can further comprise antimicrobial agents for deactivating microorganisms on surfaces, in the air, or both.

Diluents suitable for use with the present technology include, for example, water, alcohols, glycols and other organic solvents. Water is a preferred diluent. Diluents can be utilized alone or in combination with one another without departing from the spirit and scope of the present described technology.

The presently described technology provides for an allergen deactivating formulation with a low toxicity level, suitable for use in contact with human skin or animal fur and/or skin. In at least one aspect of this embodiment, an allergen deactivating formulation is provided that will not cause staining on textiles or other surfaces. Further, allergen deactivating formulations of this embodiment preferably have a low, reduced or zero level of particulates, as described above.

### DETAILED DESCRIPTION OF THE INVENTION

In one embodiment, an allergenic protein deactivating compound comprises at least about 0.2% by weight, of a quaternary amine salt mixture comprising: at least about 32% n-alkyl (50% C14, 40% C12, 10% C16) dimethyl benzyl ammonium chloride, at least about 24% n-octyl decyl dimethyl ammonium chloride, at least about 12% di-n-octyl dimethyl ammonium chloride, and at least about 12% di-n-decyl dimethyl ammonium chloride.

It is known that deposition of additives can cause streaking and filming on hard, smooth surfaces and can also cause an increased tendency towards soiling on textile surfaces. Hence, levels of allergenic protein deactivating agents which avoid or substantially reduce these undesirable side effects, while still remaining efficacious at deactivating the allergenic proteins on the surface in question, are preferred. Formulations of the presently described technology thus preferably utilize as low a level of the above agents in the final allergenic protein deactivation formulation as will be effective, in order to avoid excessive deposition of the allergenic protein deactivating compound onto the surface being treated. It is desired that the concentration of the allergenic deactivating agent, or blend of agents, be about 5% by weight, or less than about 5% by weight in the solvent, aqueous composition, or diluent used to deliver the agent or agents. Preferably, the concentration of the allergenic deactivating agent or blend of agents is less than about 2.5% by weight, less than about 2% by weight, or less than about 1.5% by weight. Most preferably, the concentration of the allergenic deactivating agent or blend of agents is about 1% by weight, or less than about 1% by weight. Suitable amounts of an allergenic deactivating agent or blend of agents that are less than about 1% include, for example, concentrations up to about 0.5% by weight, or up to about 0.2% by weight.
Although mot wanting to be bound by any particular theory, it is believed that quaternary ammonium salts of the presently described technology utilized in at least one method of the present invention may operate in a similar manner and that they may denature allergenic proteins on textiles and surfaces by chemically associating with the allergenic proteins to change their surface chemistry from protein-like to hydrocarbon-like.

Further, it is believed that the complexed allergenic proteins may remain on the textile or surface, but do not readily activate antibodies in humans because their molecular shapes have been altered in a process known as denaturation (see, A.L. Lehninger, Biochemistry, 1975, Worth Publishers, page 62, for a description of protein denaturation). It is known that antibodies or immunoglobulins form in the blood serum in response to the introduction of a foreign protein. In the case of allergenic proteins, the immunoglobulin formed is known as IgE, or Immunoglobulin E. People with allergies produce an excess of IgE, which collects around certain types of cells in the body. When people with allergies are exposed to allergenic proteins, IgE forms an antigen-antibody complex, which causes the release of chemical such as histamine from the nearby cells. These chemicals, in turn, can cause runny noses, bronchial spasms, asthma, itching, hives, nausea and even anaphylactic shock and death. Hence, it is also believed that if the surface or shape of an allergenic protein can be changed by complexation with the allergenic protein deactivating compounds of the presently described technology, then the ability of the allergenic protein to activate antibodies is reduced or negated.

In some embodiments, protein deactivating formulations of the present technology further comprise at least one antimicrobial agent. The term antimicrobial agent as used herein refers to an agent that deactivates microorganisms, and renders them inactive and/or ineffective with respect to their harmful impact in humans or animals. The term antimicrobial agent encompasses both partial and total deactivation, as well as a reduction in or a prevention of harmful impact by the microorganisms. The term antimicrobial agent also encompasses partial or total destruction of microorganisms as well as partial or total inhibition of the growth of microorganisms. Examples of types of antimicrobial agents include, but are not limited to disinfectants, sanitizers and antiseptics. The types of microorganisms deactivated by antimicrobial compounds can be spores, vegetative microorganisms, or a combination of both.

In some embodiments, antimicrobial agents suitable for use with the present technology are air treating antimicrobial agents. Air treating antimicrobial agents can be used, for example, in spray applications, to deactivate microorganisms in the air. In such applications, allergenic deactivating formulations of the present technology containing air treating antimicrobial agents can be dispensed, for example, by aerosolizing droplet particles through an actuator valve. Glycols are a particularly preferred category of air treating antimicrobial agents. Some examples of glycols suitable for use with the present technology include, but are not limited to, propylene glycol, dipropylene glycol, triethylene glycol, derivatives thereof, and mixtures thereof.

In other embodiments, antimicrobial agents suitable for use with the present technology are surface treating antimicrobial agents. Surface treating antimicrobial agents suitable for use with the present technology include antimicrobial agents for use on textiles, leather, fur, non-wovens, wood and other hard surfaces, and soft or porous surfaces. Some examples of surface treating antimicrobial agents suitable for use with the present technology include, but are not limited to, synthetic detergents such as quaternary ammonium compounds and sodium alkylbenzene sulfonates, mercury compounds, halogens, halogen compounds, aldehydes, phenolics, isothiazonlines, alcohols, carbamates, halide compounds, peroxides, parabens, iodine, metals, peracids, carbonates, hypochlorites, chloramines, benzalkonium chloride, derivatives thereof, and mixtures thereof. In some preferred embodiments, the surface treating antimicrobial agents are quaternary ammonium salts such as quaternary ammonium chlorides and quaternary ammonium saccharinates, metal salts such as silver salts, or alcohols such as isopropanol.

Allergenic protein deactivating formulations of the present technology comprise at least one allergenic protein deactivating compound. In certain embodiments, allergenic protein deactivating formulations of the present technology comprise at least one allergenic protein deactivating compound and at least one antimicrobial agent. In some preferred embodiments, allergenic protein deactivating formulations comprise at least one allergenic protein deactivating compound and at least one air treating antimicrobial agent. In other preferred embodiments, allergenic protein deactivating formulations comprise at least one allergenic protein deactivating compound and at least one surface treating antimicrobial agent. In still other preferred embodiments, allergenic protein deactivating formulations comprise at least one allergenic protein deactivating compound, at least one surface treating antimicrobial agent, and at least one air treating antimicrobial agent.

Diluents suitable for use with the present technology include, for example, water, alcohols, glycols and other organic solvents. Water is a particularly preferred diluent. Diluents can be utilized alone or in combination with one another without departing from the spirit and scope of the present described technology.

It is anticipated that the efficacy of the allergenic protein deactivating formulation(s) of the presently described technology may be improved when further combined with additives such as, for example, known allergenic protein deactivating substances, surfactants, buffers, oils and waxes, solvents, preservatives or mixtures thereof.

Examples of surfactant classes suitable for use with the present technology include, for example, nonionic surfactants, cationic surfactants, amphoteric surfactants, anionic surfactants, and combinations thereof. Although many of the compounds of the presently described technology are surfactants, the use of a secondary surfactant along with the allergen deactivating agents of the present technology is anticipated to provide improved wetting and penetration of surfaces onto which allergens might be absorbed. Improved wetting and penetration of surfaces and textiles will allow for improved delivery of the allergenic deactivating agent to the allergen.

Examples of nonionic surfactants include, for example, alcohol ethoxylates, alkylphenol ethoxylated, ethylene oxide/propylene oxide block copolymers, alkyl polyglycosides, alkanol amides, amine ethoxylates, quaternary ammonium compounds, amine oxides, polyamines, alkoxylated silicones, and combinations thereof.

Examples of anionic surfactants include, but are not limited to, linear alkylbenzene sulfonates, alcohol sulfates, alcohol ether sulfates, metal salts of long chain fatty acids, secondary alkane sulfonates, alpha olefin sulfonates, methyl ester sulfonates, phosphate esters, dialkyl sulfosuccinates, alkyl diphenyl oxide disulfonates, naphthalene sulfonates, lignosulfonates, and combinations thereof.

Examples of cationic surfactants include, for example, quaternary alkyl amines, polyquaterniums, quaternary aromatic or heterocyclic amines, and combinations thereof.

Examples of amphoteric surfactants include, for example, betaines, amine oxides, sultaines, and combinations thereof.

Known allergenic protein deactivating substances with which the current technology may be combined to produce additive or synergistic allergenic protein deactivation against multiple allergenic protein types include, but are not limited to: cyclodextrins, soluble aluminum salts, urea, alkyl trimethyl ammonium salts, polyvinyl pyrrolidone, tannic acid, immobilized tannic acid, polyphenols, enzymes, benzyl benzoate, inorganic salts, derivatives thereof, and combinations thereof.

One use of buffers in the present technology is to maintain a pH in a desirable range in formulations for the purpose of stability, solubility or efficacy. Buffers are defined as a solution containing both a weak acid and its conjugate weak base. There are hundreds of existing buffer systems. A few examples of buffer systems include, for example, triethylammonium bicarbonate, triethylammonium formate, acetate/acetic acid, carbonate/bicarbonate, potassium phosphate/phosphoric acid, and ammonium chloride/ammonium hydroxide.

Oils and waxes may be used in furniture polish compositions of the present technology to aid in dust removal from surfaces and to help maintain a glossy appearance on hard surfaces. Examples of waxes include without limitation hydrocarbon materials which have crystallinity at room temperature, natural and synthetic waxes, ester-type waxes, microcrystalline waxes, petroleum waxes, polyethylenes, polypropylenes, candililla wax, and camuba wax. Examples of oils include, for example, mineral oils, vegetable oils, linseed oil, tung oil, soybean oil, olive oil, sunflower seed oil, castor oil, canola oil, corn oil, cottonseed oil, lemon oil, orange oil, silicone oil, polydialkyl siloxanes, oligomers and polymers of alpha olefins, paraffin oil, and esters of fatty acids.

Solvents may be used with the present technology as diluents, solubilizing agents, and as a means to provide a convenient liquid delivery. Solvents used for the practice of the presently described technology are preferably liquid under ambient conditions. Examples of suitable solvents include, for example, water, methanol, ethanol, isopropanol, alcohols, ethers, esters, amides, alkanes, halogenated hydrocarbons, silicones, oils and the like.

Preservatives may be used with the present technology to provide biocidal activity in formulations of the present technology to prevent growth of mold, bacteria, fungus, and other undesirable biological substances during storage, prevent oxidation or degradation of the product and extend the useful life of the final product. Examples of preservatives include, but are not limited to, formaldehyde, antioxidants, biocides, aldehydes, fungicides, chlorinated hydrocarbons, organometallics, metal salts, organic sulfur compounds, phenolics, quaternary ammonium compounds, and combinations thereof.

All documents, e.g., patents and journal articles, cited above and/or below, are hereby incorporated by reference in their entirety.

Certain embodiments of the presently described technology are illustrated in the following examples, which are not to be construed as limiting the invention or scope of the methods or compositions described herein. One skilled in the art will recognize that modifications may be made in the presently described technology without deviating from the spirit or scope of the invention. All levels and ranges, temperatures, results, etc., used and/or described herein are approximations unless otherwise specified. Additionally, all percentages are weight percentages unless otherwise specified.

The following Example demonstrates some of the advantages of deactivating allergenic protein content using several types of allergenic protein deactivating compounds.

### Example

The allergens used in this experiment were obtained from dust mite and cockroach sources. Purified standard was used for the dust mite allergen. The cockroach allergen consisted of homogenized cockroach abdomen extracted with 5 ml of phosphate buffered saline-Tween 20 (PBS-T) extraction buffer. For each allergen, 20 microliters of allergen was placed on glass wool and allowed to coat the fibers. After application of the allergen, 60 microliters of test product was added to the glass wool and allowed to coat the fibers. Finally, 80 microliters of PBS-T was added to extract the allergen. Comparative Composition A was prepared by adding 60 microliters of sterile deionized water instead of test product prior to extraction.

Composition 1 consisted of 2% by weight of a quaternary amine salt mixture in water. The quaternary amine salt mixture consisted of:
- 32%: n-alkyl (50% C14, 40% C12, 10% C16) dimethyl benzyl ammonium chloride;
- 24%: n-octyl decyl dimethyl ammonium chloride;
- 12%: di-n-octyl dimethyl ammonium chloride;
- 12%: di-n-decyl dimethyl ammonium chloride; and
- 20%: inert ingredients.

The ELISA method for quantification of allergens in micrograms of allergen per ml of sample was then conducted for each extract to determine whether the test products resulted in a deactivation of allergenic proteins. The results are shown in Table 1 below.

**Table 1**

| | Dust mite DER p1 mcg/ml | Cockroach Bla g1 U/ml |
|---|---|---|
| Comparative Composition A | 2.86 | 4.00 |
| Composition 1 | 2.96 | 1.13 |

Composition 1 provided deactivation of cockroach allergenic protein.

The presently described technology and the manner and process of making and using it, are now described in such full, clear, concise and exact terms as to enable one of ordinary skill in the art to which the present technology pertains, to make and use the same. It should be understood that the foregoing describes some embodiments and advantages of the invention and that modifications may be made therein without departing from the spirit and scope of the presently described technology as set forth in the claims. Moreover, the present technology has been described with reference to preferred and alternate embodiments. Modifications and alterations will occur to others upon the reading and understanding of the specification. It is intended to include all such modifications and alterations insofar as they come within the scope of the appended claims or equivalents thereof. To particularly point out and distinctly claims the subject matter regarded as the invention, the following claims conclude this specification.

## Claims

1. An allergenic protein deactivating formulation comprising an allergenic protein deactivating compound comprising at least about 0.05% by weight of a quaternary amine salt mixture comprising:
at least about 32% by weight n-alkyl (50% C14, 40% C12, 10% C16) dimethyl benzyl ammonium chloride;
at least about 24% by weight n-octyl decyl dimethyl ammonium chloride;
at least about 12% by weight di-n-octyl dimethyl ammonium chloride; and
at least about 12% by weight di-n-decyl dimethyl ammonium chloride.

2. The allergenic protein deactivation formulation of claim 1, further comprising at least one antimicrobial agent.

3. The allergenic protein deactivation formulation of claim 2, wherein the at least one antimicrobial agent is a surface treating antimicrobial agent.

4. The allergenic protein deactivation formulation of claim 3, wherein the surface treating antimicrobial agent is selected from the group consisting of quaternary ammonium compounds, sodium alkylbenzene sulfonates, mercury compounds, halogens, halogen compounds, aldehydes, phenols, isothiazonlines, alcohols, carbamates, halide compounds, peroxides, parabens, iodine, metals, peracids, carbonates, hypochlorites, chloramines, benzalkonium chloride, derivatives thereof, and mixtures thereof.

5. The allergenic protein deactivation formulation of claim 2, wherein the at least one antimicrobial agent is an air treating antimicrobial agent.

6. The allergenic protein deactivation formulation of claim 5, wherein the air treating antimicrobial agent is selected from the group consisting of propylene glycol dipropylene glycol, triethylene glycol, derivatives thereof, and mixtures thereof.

7. A method for deactivating allergenic proteins on a surface, in the air or on skin comprising the step of exposing the surface, air or skin to an allergenic protein deactivating formulation which comprises an allergenic protein deactivating compound comprising at least about 0.05% by weight of a quaternary amine salt mixture comprising:
at least about 32% by weight n-alkyl (50% C14, 40% C12, 10% C16) dimethyl benzyl ammonium chloride;
at least about 24% by weight n-octyl decyl dimethyl ammonium chloride;
at least about 12% by weight di-n-octyl dimethyl ammonium chloride; and
at least about 12% by weight di-n-decyl dimethyl ammonium chloride.

8. The method of claim 7, wherein the allergenic protein deactivating compound further comprises at least one antimicrobial agent.

9. The method of claim 8, wherein the at least one antimicrobial agent is a surface treating antimicrobial agent.

10. The method of claim 9, wherein the surface treating antimicrobial agent is selected from the group consisting of quaternary ammonium compounds, sodium alkylbenzene sulfonates, mercury compounds, halogens, halogen compounds, aldehydes, phenols, isothiazonlines, alcohols, carbamates, halide compounds, peroxides, parabens, iodine, metals, peracids, carbonates, hypochlorites, chloramines, benzalkonium chloride, derivatives thereof, and mixtures thereof.

11. The method of claim 8, wherein the at least one antimicrobial agent is an air treating antimicrobial agent.

12. The method of claim 8, wherein the air treating antimicrobial agent is selected from the group consisting of propylene glycol, dipropylene glycol, triethylene glycol, derivatives thereof, and mixtures thereof.

## Patentansprüche

1. Allergene Protein-inaktivierende Formulierung, die eine allergene Protein-inaktivierende Verbindung umfasst, die mindestens etwa 0,05 Gew.-% eines quaternären Aminsalzgemisches umfasst, das umfasst:
mindestens etwa 32 Gew.-% n-Alkyl (50% C14, 40% C12, 10% C16) dimethylbenzylammoniumchlorid,
mindestens etwa 24 Gew.-% n-Octyldecyldimethylammoniumchlorid,
mindestens etwa 12 Gew.-% Di-n-octyldimethylammoniumchlorid und
mindestens etwa 12 Gew.-% Di-n-decyldimethylammoniumchlorid.

2. Allergene Protein-inaktivierende Formulierung nach Anspruch 1, die ferner mindestens ein antimikrobielles Mittel umfasst.

3. Allergene Protein-inaktivierende Formulierung nach Anspruch 2, wobei das mindestens eine antimikrobielle Mittel ein antimikrobielles Mittel zur Oberflächenbehandlung ist.

4. Allergene Protein-inaktivierende Formulierung nach Anspruch 3, wobei das antimikrobielle Mittel zur Oberflächenbehandlung ausgewählt ist aus der Gruppe, bestehend aus quaternären Ammoniumverbindungen, Natriumalkylbenzolsulfonaten, Quecksilberverbindungen, Halogenen, Halogenverbindungen, Aldehyden, Phenolen, Isothiazonlinen, Alkoholen, Carbamaten, Halogenidverbindungen, Peroxiden, Parabenen, Iod, Metallen, Persäuren, Carbonaten, Hypochloriten, Chloraminen, Benzalkoniumchlorid, Derivaten davon und Gemischen davon.

5. Allergene Protein-inaktivierende Formulierung nach Anspruch 2, wobei das mindestens eine antimikrobielle Mittel ein antimikrobielles Mittel zur Luftbehandlung ist.

6. Allergene Protein-inaktivierende Formulierung nach Anspruch 5, wobei das antimikrobielle Mittel zur Luftbehandlung ausgewählt ist aus der Gruppe, bestehend aus Propylenglykol, Dipropylenglykol, Triethylenglykol, Derivaten davon und Gemischen davon.

7. Verfahren zum Inaktivieren von allergenen Proteinen auf einer Oberfläche, in der Luft oder auf Haut, das den Schritt einer Exponierung der Oberfläche, der Luft oder der Haut gegenüber einer allergenen Protein-inaktivierenden Formulierung umfasst, die eine allergene Protein-inaktivierende Verbindung umfasst, die mindestens etwa 0,05 Gew.-% eines quaternären Aminsalzgemisches umfasst, das umfasst:
mindestens etwa 32 Gew.-% n-Alkyl (50% C14, 40% C12, 10% C16) dimethylbenzylammoniumchlorid,
mindestens etwa 24 Gew.-% n-Octyldecyldimethylammoniumchlorid,
mindestens etwa 12 Gew.-% Di-n-octyldimethylammoniumchlorid und
mindestens etwa 12 Gew.-% Di-n-decyldimethylammoniumchlorid.

8. Verfahren nach Anspruch 7, wobei die allergene Protein-inaktivierende Verbindung ferner mindestens ein antimikrobielles Mittel umfasst.

9. Verfahren nach Anspruch 8, wobei das mindestens eine antimikrobielle Mittel ein antimikrobielles Mittel zur Oberflächenbehandlung ist.

10. Verfahren nach Anspruch 9, wobei das antimikrobielle Mittel zur Oberflächenbehandlung ausgewählt ist aus der Gruppe, bestehend aus quaternären Ammoniumverbindungen, Natriumalkylbenzolsulfonaten, Quecksilberverbindungen, Halogenen, Halogenverbindungen, Aldehyden, Phenolen, Isothiazonlinen, Alkoholen, Carbamaten, Halogenidverbindungen, Peroxiden, Parabenen, Iod, Metallen, Persäuren, Carbonaten, Hypochloriten, Chloraminen, Benzalkoniumchlorid, Derivaten davon und Gemischen davon.

11. Verfahren nach Anspruch 8, wobei das mindestens eine antimikrobielle Mittel ein antimikrobielles Mittel zur Luftbehandlung ist.

12. Verfahren nach Anspruch 8, wobei das antimikrobielle Mittel zur Luftbehandlung ausgewählt ist aus der Gruppe, bestehend aus Propylenglykol, Dipropylenglykol, Triethylenglykol, Derivaten davon und Gemischen davon.

## Revendications

1. Formulation pour la désactivation des protéines allergisantes, comprenant un composé désactivant les protéines allergisantes comprenant au moins environ 0,05% en poids d'un mélange de sels d'amine quaternaires comprenant:
au moins environ 32% en poids de chlorure de n-alkyl (50% en C14, 40 % en C12, 10% en C16) diméthyl benzyl ammonium;
au moins environ 24% en poids de chlorure de n-octyl décyl diméthyl ammonium;
au moins environ 12% en poids de chlorure de di-n-octyl diméthyl ammonium; et
au moins environ 12% en poids de chlorure de di-n-décyl diméthyl ammonium.

2. Formulation pour la désactivation des protéines allergisantes selon la revendication 1, comprenant en outre au moins un agent antimicrobien.

3. Formulation pour la désactivation des protéines allergisantes selon la revendication 2, dans laquelle le au moins un agent antimicrobien est un agent antimicrobien pour le traitement des surfaces.

4. Formulation pour la désactivation des protéines allergisantes selon la revendication 3, dans laquelle l'agent antimicrobien pour le traitement des surfaces est choisi dans le groupe consistant en composés d'ammonium quaternaires, alkylbenzène sulfonates de sodium, composés de mercure, halogènes, composés d'halogènes, aldéhydes, phénols, isothiazolines, alcools, carbamates, composés d'halogénures, peroxydes, parabens, iode, métaux, peracides, carbonates, hypochlorites, chloramines, chlorure de benzalkonium, dérivés de ceux-ci, et mélanges de ceux-ci.

5. Formulation pour la désactivation des protéines allergisantes selon la revendication 2, dans laquelle le au moins un agent antimicrobien est un agent antimicrobien pour le traitement de l'air.

6. Formulation pour la désactivation des protéines allergisantes selon la revendication 5, dans laquelle l'agent antimicrobien pour le traitement de l'air est choisi dans le groupe consistant en propylène glycol, dipropylène glycol, triéthylène glycol, dérivés de ceux-ci, et mélanges de ceux-ci.

7. Procédé pour la désactivation des protéines allergisantes sur une surface, dans l'air ou sur la peau, comprenant l'étape d'exposition de la surface, de l'air ou de la peau à une formulation pour la désactivation des protéines allergisantes qui comprend un composé de désactivation des protéines allergisantes comprenant au moins environ 0,05% en poids d'un mélange de sels d'amine quaternaires comprenant:
au moins environ 32% en poids de chlorure de n-alkyl (50% en C14, 40% en C12,10% en C16) diméthyl benzyl ammonium;
au moins environ 24% en poids de chlorure de n-octyl décyl diméthyl ammonium;
au moins environ 12% en poids de chlorure de di-n-octyl diméthyl ammonium; et
au moins environ 12% en poids de chlorure de di-n-décyl diméthyl ammonium.

8. Procédé selon la revendication 7, dans lequel le composé de désactivation des protéines allergisantes comprend en outre au moins un agent antimicrobien.

9. Procédé selon la revendication 8, dans lequel le au moins un agent antimicrobien est un agent antimicrobien pour le traitement des surfaces.

10. Procédé selon la revendication 9, dans lequel l'agent antimicrobien pour le traitement des surfaces est choisi dans le groupe consistant en composés d'ammonium quaternaires, alkylbenzène sulfonates de sodium, composés de mercure, halogènes, composés d'halogènes, aldéhydes, phénols, isothiazolines, alcools, carbamates, composés d'halogénures, peroxydes, parabens, iode, métaux, peracides, carbonates, hypochlorites, chloramines, chlorure de benzalkonium, dérivés de ceux-ci, et mélanges de ceux-ci.

11. Procédé selon la revendication 8, dans lequel le au moins un agent antimicrobien est un agent antimicrobien pour le traitement de l'air.

12. Procédé selon la revendication 8, dans lequel l'agent antimicrobien pour le traitement de l'air est choisi dans le groupe consistant en propylène glycol, dipropylène glycol, triéthylène glycol, dérivés de ceux-ci, et mélanges de ceux-ci.
